(19) Europäisches Patentamt
European Patent Office
Office européen des brevets

(11) **EP 2 011 437 A1**

(12) **EUROPEAN PATENT APPLICATION**

(43) Date of publication:
**07.01.2009 Bulletin 2009/02**

(51) Int Cl.:
***A61B 5/053*** (2006.01)

(21) Application number: **08159566.2**

(22) Date of filing: **02.07.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **03.07.2007 JP 2007174699**

(71) Applicant: **TANITA CORPORATION Tokyo 174-8630 (JP)**

(72) Inventor: **Watanabe, Moto Tokyo 174-8630 (JP)**

(74) Representative: **Grünecker, Kinkeldey, Stockmair & Schwanhäusser Anwaltssozietät Leopoldstrasse 4 80802 München (DE)**

(54) **A body composition measuring device, a body composition measuring method**

(57) The body composition measuring device and method which can perform measurement of the value about body composition also to those who have a deficit in the limbs are provided.

A plurality of current applicators and voltage measurement electrodes are provided, it is a body composition measuring device which measures body composition by bioelectricity impedance measurement, and body composition is measured from a value of bioelectricity impedance measurement using a plurality of regression.

A plurality of electrodes for current application and voltage measurement electrodes are made to correspond to left hand, right hand, left foot, and right foot for a being-measured person.

In addition, a plurality of regression is made to correspond to a value of bioelectricity impedance measurement of an electrode corresponding to between both feet, between both hands, right half body, left half body, right hand, left hand, right foot, left foot, and trunk.

| CASE | RIGHT HAND | LEFT HAND | RIGHT FOOT | LEFT FOOT | |
|---|---|---|---|---|---|
| 1. | ○ | ○ | ○ | ○ | REGRESSION FOR LEFT HALF BODIES (LHF) |
| 5. | ○ | ○ | × | ○ | |
| 2. | × | ○ | ○ | ○ | |
| 10. | × | ○ | × | ○ | |
| 6. | ○ | ○ | ○ | × | USE DATA BETWEEN RIGHT HAND AND RIGHT FOOT FOR REGRESSION OF LEFT HALF BODY (LHF) |
| 3. | ○ | × | ○ | ○ | |
| 11. | ○ | × | ○ | × | |
| 9. | ○ | × | × | ○ | USE DATA BETWEEN RIGHT HAND AND LEFT FOOT FOR REGRESSION OF LEFT HALF BODY (LHF) |
| 8. | × | ○ | ○ | × | USE DATA BETWEEN LEFT HAND AND RIGHT FOOT FOR REGRESSION OF LEFT HALF BODY (LHF) |
| 4. | × | × | ○ | ○ | USE DATA BETWEEN BOTH FEET FOR REGRESSION (FF) |
| 7. | ○ | ○ | × | × | USE DATA BETWEEN BOTH HANDS FOR REGRESSION (HH) |

※ BY TOUCHING ELECTRODE, SELECT REGRESSION AND COMPUTE.

**Fig. 11**

**Description**

CROSS REFERENCE OF THE INVENTION

The present application claims benefit of the filing date of Japanese Patent Application No. 2007-174699 filed July 03, 2007, and the contents of which are herein incorporated by reference in their entirety.

BACKGROUND OF THE INVENTION

[Field of the Invention]

Especially, this invention relates to a body composition measuring device and a body composition measuring method.

[Background of the Invention]

In recent years, demand to measure body composition, such as a body fat ratio, in addition to weight is increasing by the rise of a health aim.
In order to know the body composition, it is convenient and exact to measure bioelectricity impedance.
By measurement of bioelectricity impedance, body composition can be measured by utilizing the difference of electrical conductivity from the adipose tissue and the body water containing electrolyte in the body.
As this bioelectricity impedance measuring device, an apparatus of four electrodes which are the pairs of an electrode for current application and an electrode for voltage measurement is used formerly.
In the bioelectricity impedance measuring device of these four electrodes, current is supplied from electrodes at the side of tiptoes of both feet, and the voltage is measured at the heel side.
In this time, current flows from one leg into another leg via the abdomen.
Thereby, bioelectricity impedance to measure is impedance between both feet.
This result of bioelectricity impedance measurement between both feet is applied to one regression, and body composition (for example, body fat ratio) of the whole body is presumed.
On the other hand, in order to measure more exact body composition, a bioelectricity impedance measuring device of eight electrodes gains popularity.
As a bioelectricity impedance measuring device of eight electrodes, for example to refer a patent documents 1, there is a biometry equipment of eight electrodes which measures by changing the electrodes and displays a part of the measured body (it is hereafter considered as conventional technology 1.).

[Patent documents 1] JP,2001-178696,A

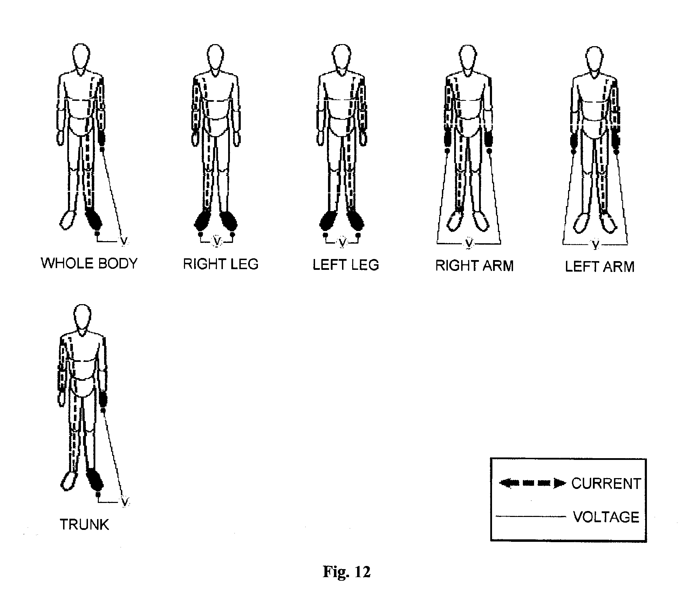

When it explains with reference to Fig. 12, in a bioelectricity impedance measuring device of eight electrodes of conventional technology 1, current is supplied from electrodes of the tiptoe side of both feet and the fingertip side of both hands, and voltage is measured by the heel side of both feet and the thenar side of both hands.
For this bioelectricity impedance measuring device of eight electrodes, bioelectricity impedance measurement of the whole body and each part (a right leg, a left leg, a right arm, a left arm, and a trunk) are carried out by changing a part which applies current and a position which measures voltage.
For example, when measuring impedance of right leg, current is sent between right-hand and right leg, and voltage between both feet is measured. Similarly, by switching the eight electrodes, a part which applies current, and a part which makes voltage measurement can be changed as shown in Fig. 12, and bioelectricity impedance of a right leg, a left leg, a right arm, a left arm, a trunk, and a left side of the body can be measured.
Further, body composition of the whole body based on bioelectricity impedance of the left side of the body can be presumed.

[Description of the Invention]

[Problem(s) to be Solved by the Invention]

However, the biometry equipment of conventional technology 1 has the problem that body composition can not be measured if the electrodes do not touch all the hands and the feet.
This is because body composition of the whole body is measured by one regression on condition of each part of the body existing in the bioelectricity impedance measuring device of eight electrodes as well as the bioelectricity impedance measuring device of four electrodes.

For this reason, body composition of the whole body can not be measured accurately for a subject who lacks one arm, one leg, both arms, or both legs and a being-measured person who wear a plaster cast or the like by injury and can not touch all the electrodes with the skins.

This invention is made in view of such a situation, and makes it a subject to cancel an above-mentioned subject.

SUMMARY OF THE INVENTION

A body composition measuring device of this invention measures bioelectricity impedance from measured value measured by using electrodes for current application and voltage measurement electrodes and providing with a calculator which determines body composition from this bioelectricity impedance by using these electrodes; and the calculator determines body composition by using regression of the bioelectricity impedance corresponding to each of between both feet, between both arms, left half body, right half body, left leg, right leg, left arm, right arm, and trunk.

A body composition measuring device of this invention is a plurality of the electrodes for current application and voltage measurement electrodes are provided for being-measured person corresponding to left arm, right arm, left leg, and right leg.

A body composition measuring device of this invention is an electrode for current application and a voltage measurement electrode; in which are provided for the being-measured person corresponding to left arm, right arm, left leg, and right leg, body composition is computed by using regressions corresponding to an electrode which is not used for a measurement of the bioelectricity impedance by using other electrodes.

A body composition measuring device of this invention measures body composition by using the regression corresponding to the left arm and / or left leg, when not using an electrode corresponding to right arm and / or right leg.

A body composition measuring device of this invention, when not using an electrode corresponding to left arm and /or left leg, uses a value of the bioelectricity impedance measurement of an electrode corresponding to right arm and / or right leg for the regression corresponding to left arm and / or left leg and measures body composition.

A body composition measuring device of this invention, when not using an electrode corresponding to left arm and right leg, uses a value of the bioelectricity impedance measurement of an electrode corresponding to right arm and left leg for the regression corresponding to left arm and left leg and measures body composition.

A body composition measuring device of this invention, when not using an electrode corresponding to right arm and left leg, uses a value of the bioelectricity impedance measurement of an electrode corresponding to left arm and right leg for the regression corresponding to left arm and left leg and measures body composition.

A body composition measuring device of this invention carries out equalization or removal of an abnormal value for a value of body composition determined from a plurality of regression.

A body composition measuring device of this invention measures body composition of a part where an electrode is used for the body composition in addition to body composition of whole body.

A body composition measuring method of this invention measures bioelectricity impedance by using an electrode for current application and a voltage measurement electrode; and calculator determines body composition from this bioelectricity impedance, and body composition is measured by using regression of the bioelectricity impedance corresponding to each of between both feet, between both arms, right side of the body, left side of the body, right leg, left leg, right arm, left arm, and trunk.

[Effect of the Invention]

According to this invention, a body composition measuring device that measures accurately body composition of a being-measured person without one arm, one leg, both arms, or both legs can be provided by having a plurality of regression.

Also, a body composition measuring method which measures accurately body composition of a being-measured person without one arm, one leg, both arms, or both legs can be provided by using a plurality of regression.

BRIEF DESCRIPTION OF THE DRAWINGS

Fig. 1 is a conceptual diagram indicating the external composition of a measuring device related to an embodiment of the invention.

Fig. 2 is a block diagram indicating the internal configuration of the measuring device related to an embodiment of the invention.

Fig. 3 is a flow chart showing the measurement procedure of a measuring device and the outline of operation related to an embodiment of the invention.

Fig 4. is a representative circuit schematic showing bioelectricity impedance.

Fig. 5 is a graph charts showing a bioelectricity impedance vector locus.

Fig. 6 is a graph that indicates correlation with the body fat ratio of whole body determined from each part of the body of the man related to an embodiment of the invention and the body fat ratio determined by the DXA method.

Fig. 7 is the graph that indicates correlation with the body fat ratio of whole body determined from each part of the body of the woman related to an embodiment of the invention and the body fat ratio determined by the DXA method.

Fig. 8A and 8B are conceptual diagrams indicating the part and measuring method in case of measuring the body composition related to an embodiment of the invention.

Fig. 9 is a conceptual diagram indicating the part and measuring method in case of measuring the body composition related to an embodiment of the invention.

Fig. 10 is a conceptual diagram indicating the part and measuring method in case of measuring the body composition related to an embodiment of the invention.

Fig. 11 is a conceptual diagram indicating the main regression for measuring the body composition of the whole body related to an embodiment of the invention.

Fig. 12 is a conceptual diagram indicating the conventional measuring method by the bioelectricity impedance measuring device of eight electrodes.

DESCRIPTION OF THE PREFERRED EMBODIMENTS

[Best Mode of Carrying Out the Invention]

<Embodiment>

(Composition of a body composition measuring device)

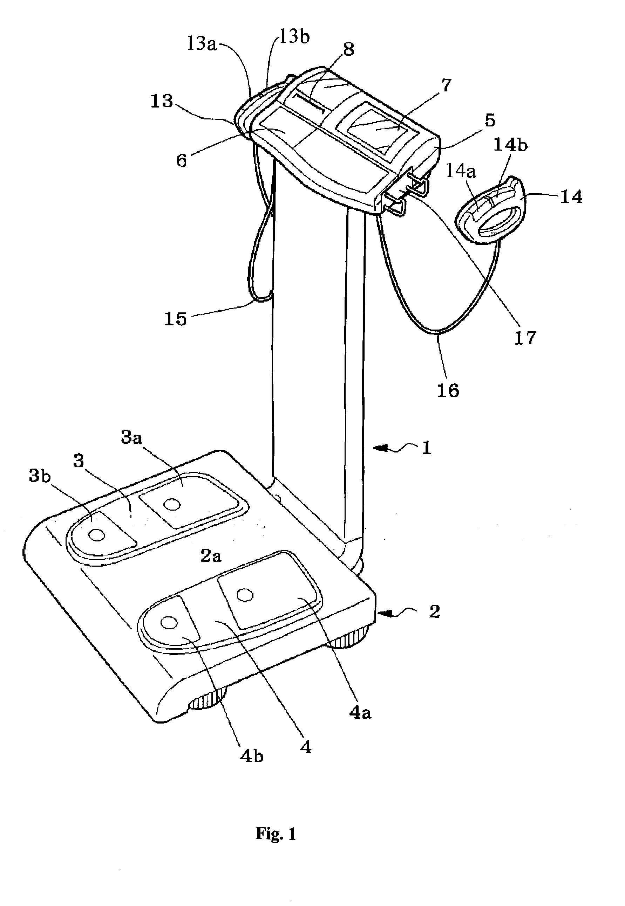

Fig. 1 is an appearance perspective view of a body composition measuring device which is one example of this invention, and measuring device 1 forms roughly "L" shape.

Scale 2 is formed in the lower part and this scale 2 is publicly known; and loading table side 2a which appears in order to measure a subject's weight has formed foot stand 3 which the bottom of a subject's left leg touches, and foot stand 4 which the bottom of a right leg touches.

This foot stand 3 provides current applicator 3a for sending current and voltage measurement electrode 3b for measuring voltage.

Also, similarly, foot stand 4 provides current applicator 4a for sending current and voltage measurement electrode 4b for measuring voltage.

Thus, at the time of measurement of a being-measured person, current applicator 3a and voltage measurement electrode 3b contact the left foot, and current applicator 4a and voltage measurement electrode 4b contact the right foot.

Operation box 5 is provided in the upper part of measuring device 1.

This operation box 5 is comprised a plurality of keys such as a power switch and numeric keys, and it is provided that input device 6 which are input method such as a ten key which inputs various kinds of body information, display 7 which is a display method which displays a measurement result and comprises organic electroluminescence, dot matrix LCD, or the like, printing device 8 which prints a measurement result on a paper.

Further, electrode grip 13 for left hands is connected to operation box 5 via code 15.

Also, electrode grip 14 for right hand is connected via code 16.

Current applicator 13a which sends current and voltage measurement electrode 13b which measures voltage are provided in electrode grip 13 for the left hand.

Similarly, current applicator 14a which sends current and voltage measurement electrode 14b which measures voltage are provided in electrode grip 14 for the right hand.

Therefore, the skin of a palm of a left hand, current applicator 13a, and voltage measurement electrode 13b can be contacted at the time of measurement of a being-measured person.

Similarly, the skin of a palm of a right hand, current applicator 14a and voltage measurement electrode 14b can be contacted.

Electrode grip 13 for hands and electrode grip 14 are hung on hook 17 provided in both sides of left and right of operation box 5 except the time of measurement.

(Control composition)

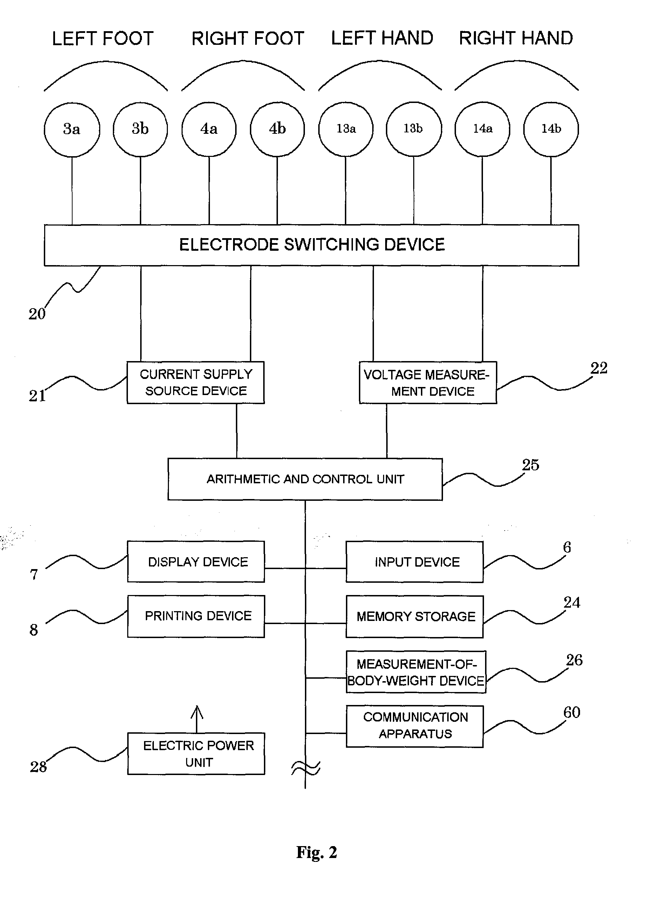

Fig. 2 is a control block diagram of measuring device 1 related to an embodiment of the invention.

Eight electrodes, current applicators 3a, 4a, 13a, and 14a, and voltage measurement electrodes 3b, 4b, 13b, and 14b, which are electrodes for current application and electrodes for voltage measurement and touch a hand and a foot on either side, are connected to electrode switching device 20, respectively.

This electrode switching device 20 is connected to arithmetic and control unit 25 which is a control means via current supply source device 21 and voltage measurement device 22.

This arithmetic and control unit 25 is provided with a central processing unit (CPU), a microcomputer, or the like; it is a bioelectricity impedance calculating means which computes bioelectricity impedance from applied current and measurement voltage; and it is also a compensation means which corrects computed bioelectricity impedance.

Further, this arithmetic and control unit 25 is also a body composition calculating means which computes the characteristic about a living body's composition, and carries out various kinds of operations, control, and others.

In this arithmetic and control unit 25 are connected to: memory storage 24 which is a memory measure that memorizes various data or the like and is a register, RAM, ROM, flash memory, or HDD, measurement-of-body-weight device 26 which measures a subject's weight, input device 6, display 7, and printing device 8.

In this arithmetic and control unit 25, communication apparatus 60 is connected, which is connectable with other computers which are PCs (personal computer) or the like and is a terminal, such as USB (universal serial bus) and LAN (local area network), or a contact of wireless LAN, Bluetooth (registered trademark), and wireless USB, or the like.

Electric power unit 28 supplies electric power to arithmetic and control unit 25 or each other devices.

(Measurement of body composition)

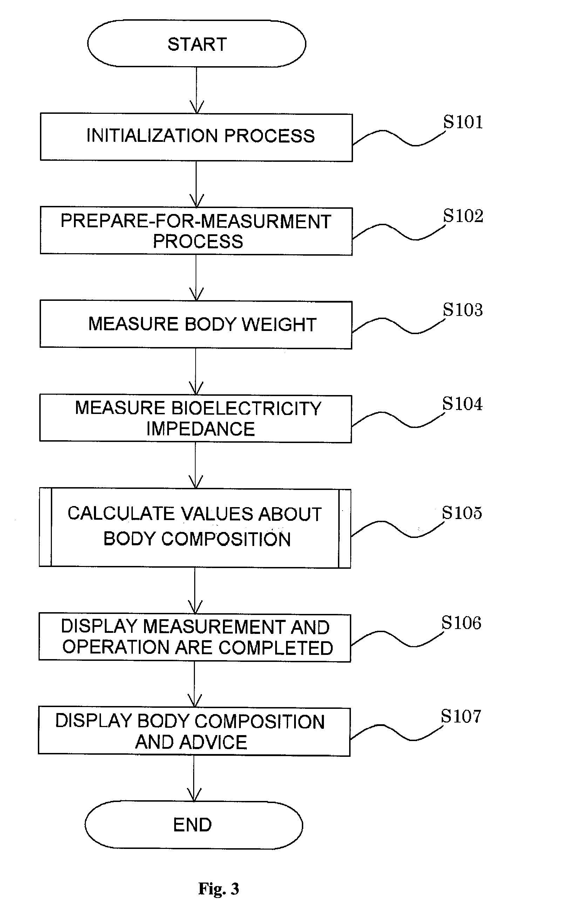

Then, measurement of the body composition related to an embodiment of the invention is explained with reference to Fig. 3, which is a flow chart.

At first, when measuring person push on a switch which does not illustrate, control source voltage to each part from electric power unit 28 is supplied.

Thereby, arithmetic and control unit 25 starts and begins to execute firmware memorized in memory storage 24's ROM, flash memory, or the like.

(Step S101)

Then, in Step S101, arithmetic and control unit 25 carries out initialization processing described in firmware. Thereby, arithmetic and control unit 25 initializes each part, and displays the initial screen which is not illustrated on display 7.

When the initialization processing of each part is completed, arithmetic and control unit 25 advances processing to the following step S102.

(Step S102)

In Step S102, arithmetic and control unit 25 displays having ended starting process and having completed the preparation-for-measurement on display 7.

Here, individual parameters, such as sex, age, height, and weight of clothes, are inputted by measuring person or the being-measured person.

The input of this individual parameter is carried out using the number key of input device 6, or the like.

Also, it may input by other computers via communication apparatus 60.

About the data of this individual parameter, arithmetic and control unit 25 memorizes to RAM of memory storage 24.

It can also be inputting again when the input of this individual parameter is wrong.

Measuring device 1 related to an embodiment of the invention can input from a menu which part is measured, when the being-measured person deficit one arm, one leg, both arms, or both-legs, or is in a plaster cast, where the electrode cannot be contacted.

Furthermore, it is also enabled to detect automatically an electrode is touched / untouched by measurement of a value of a pressure sensor, an electrostatic capacity, or the like, and to choose about which part is to be measured.

Here, when a being-measured person presses the key of input device 6 interactively and directs a measurement start according to instructions of the menu displayed on display 7, an actual measuring process is started.

(Step S103)

In Step S103, when a being-measured person rides on scale 2, measurement-of-body-weight device 26 detects a load and measures weight of the being-measured person.

About the measured weight, measurement-of-body-weight device 26 memorizes to RAM of memory storage 24.

(Step S104)

Then, in Step S104, arithmetic and control unit 25 measures bioelectricity impedance (living body BI). Firstly, the operation of the bioelectricity impedance measurement (multifrequency body impedance measurement) by using the AC of a plurality of frequency carried out in this case is explained in the following.

Multifrequency bioelectricity impedance measurement carries out n times set up from i = 1 as the frequency Fi for the first time.

Namely, as initialization of the measurement in the first frequency, i = 1 is set up, and frequency Fi is set up with the value of this "i".

Based on the gauge control parameter allocated previously at ROM in memory storage 24 (it is hereafter written as a gauge control parameter), arithmetic and control unit 25 sets up output signal frequency and outputs the output signal to current supply source device 21.

Current supply source device 21 comprises a constant current output circuit which can set up a current value, and it sets up an output current value based on the gauge control parameter.

The AC output is applied to each part of the body of a being-measured person with the current applicator switched via electrode switching device 20.

This is measured by corresponding voltage measurement electrode switched via electrode switching device 20.

At the time of this measurement, it detects the current which flows into a being-measured person from the reference resistance (not illustrated) set up separately; the analog signal of the output is converted into a digital signal by using the A/D converter in arithmetic and control unit 25; and that result is memorized to RAM of memory storage 24.

Simultaneously, it is inputted into the differential amplifier circuit in voltage measurement device 22 through the voltage measurement electrode corresponding to each part of the body contacted by the being-measured person, and this differential amplifier circuit outputs the signal which is a difference of each inputted voltage to the A/D converter in arithmetic and control unit 25.

An A/D converter measures bioelectricity impedance by converting the inputted analog signal into a digital signal and memorizes the result to RAM.

If the impedance measurement by the first frequency is completed, 1 is added to i; and it is judged whether the prescribed number of determinations is ended.

Here, if i is over n of a predetermined number, measurement of impedance is ended; and if it has not yet exceeded, it will carry out impedance measurement in the following frequency.

Then, from the bioelectricity impedance measured value measured at Step S104, a bioelectricity impedance vector locus and parameters about it are computed.

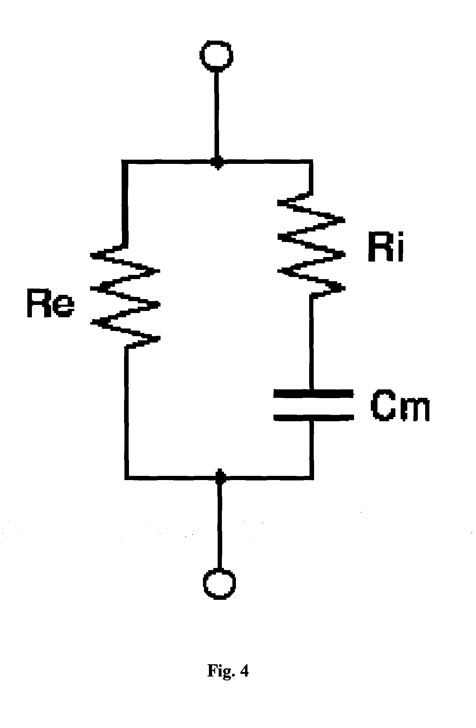

Usually, although bioelectricity impedance is expressed in an equivalent circuit that described by concentrated constants as extra cellular fluid resistance Re, intracellular fluid resistance Ri, and cell membrane capacity Cm, as shown in Fig. 4; since, in fact, each cell which comprises a living body is expressed in a circuit where constants differ by a form and difference in character, respectively; in a living body which is the collection, a bioelectricity impedance vector locus does not act as a semicircle in the case of measuring an equivalent circuit by a concentrated constant, and it is supposed that it becomes a circle according to a Cole-Cole circular arc law.

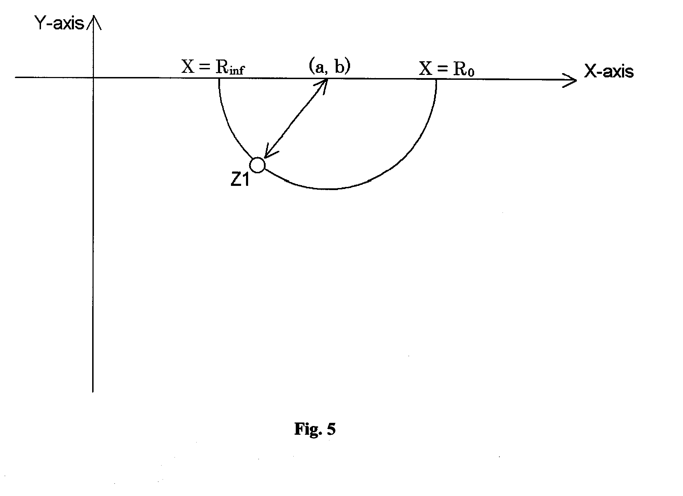

Therefore, generally, bioelectricity impedance draws a circular locus as shown in Fig. 5.

Here, the X-axis expresses a resistance component of bioelectricity impedance, and the Y-axis expresses a reactance component of bioelectricity impedance.

Because the reactance component of bioelectricity impedance is capacitive and shows a minus value, the bioelectricity impedance vector locus is located under the X-axis; also, a bioelectricity impedance measured value Z1, Z2, --, ZN in respective frequency F1, F2, --, FN is on a circumference of a certain circle by the assumption that the bioelectricity impedance vector locus to determine is a circle.

Here, when X coordinate of the center of the circle is set "a," Y coordinate of the center of the circle is set "b," radius of the circle is set "r," an equation of the circle which passes along bioelectricity impedance measured value is expressed, such as equation 1.

$$(X - a)^2 + (Y - b)^2 = r^2 \quad \dots \text{(equation 1)}$$

"a", "b", and "r" are determined by substituting bioelectricity impedance measured value Z1, Z2, --, ZN for respective frequency F1, F2, --, FN to Equation 1.

X is expressed by Equation 1, as follows.

$$X = a \pm sqrt(r^2 - b^2) \ldots (equation\ 2)$$

sqrt() indicates a square root.

Then, intersection $R_0$ and $R_{inf}$ ($R_0 > R_{inf}$) for the circle and the X-axis, which are indicated with equation 1, are calculated as follows by equation 2.

$$R_0 = a + sqrt\ (r^2 - b^2) \ldots (equation\ 3)$$

$$R_{inf} = a - sqrt\ (r^2 - b^2) \ldots (equation\ 4)$$

Re and Ri in the equivalent circuit of Fig. 4 are calculated as follows from equation 3 and equation 4.

$$Re = R_0 \qquad\qquad \ldots (equation\ 5)$$

$$Ri = R_0 * R_{inf}\ /\ (R_0 - R_{inf}) \quad \ldots (equation\ 6)$$

Since bioelectricity impedance vector Zc in characteristic frequency Fc is the point that the absolute value of a reactance component, that is, Y axial component, becomes the maximum, X coordinate value which is the resistance components and the Y coordinate value which is the reactance component in that case are computed as follows.

$$X = a \qquad \ldots (equation\ 7)$$

$$Y = b - r \qquad \ldots (equation\ 8)$$

Here, when Rc is the resistance component of Zc, and Xc is the reactance component of Zc, Zc is expressed as follows.

$$Zc = Rc + j * Xc = a + j * (b - r) \qquad \ldots (equation\ 9)$$

Also, when Z(omega) is the bioelectricity impedance vector in omega, and tau and beta are constants, the bioelectricity impedance vector in any circular frequency omega is expressed as follows by Cole-Cole circular arc law.

$$Z(omega) = (R_0 - R_{inf})\ /\ \{\ 1 + (j * omega * tau)^{beta}\} \ldots$$

$$(equation\ 10)$$

Furthermore, equation 10 is expressed as follows as tau = 1 / (omega * c).

$$Z(omega) = (R_0 - R_{inf}) / \{1 + (j * omega / omega * c)^{beta}\} \dots (equation\ 11)$$

Here, since it is omega * c = 2 * pi * Fc, Fc and beta are determined using the bioelectricity impedance measured value measured previously.

(Step S105)

Then, in Step S105, as values about body composition, arithmetic and control unit 25 calculates extracellular fluid volume, intracellular fluid volume, and the amount of body water, and memorizes to RAM of memory storage 24. This is, based on the bioelectricity impedance vector locus calculated from bioelectricity impedance measured value as mentioned above and their related parameter $R_0$ and $R_{inf}$, Re and Ri, Zc, Rc, Xc, Fc, or the like, it is computed extracellular fluid volume, intracellular fluid volume, and body water fluid volume (sum of extracellular fluid volume and intracellular fluid volume).

Then, as values about body composition, a body fat ratio, an amount of body fat, a fat-free rate, and a fat-free mass are calculated in the whole body and the each part, respectively.
The body fat ratio is determined based on parameters, such as weight, height, sex, age, resistance, reactance, and impedance.
For example, the body fat ratio is determined by using the equation prepared according to male or female, as follows:

Body fat ratio of the whole body = f(height, weight, resistance between left hand and left leg, reactance between left hand and left leg, impedance between left hand and left leg)

Amount of body fat is calculated based on the weight and the body fat ratio.
For example, the amount of body fat of whole body is calculated by the following equations:

```
Amount of body fat of the whole body = the weight * the
body fat ratio of whole body
```

The fat-free rate is determined based on the body fat ratio.
For example, the fat-free rate of whole body is determined by the following formulas:

```
Fat-free rate of whole body = 100 - body fat ratio of whole
body
```

The fat-free mass is calculated based on weight and amount of body fat.
For example, the fat-free mass of whole body is calculated by the following formulas:

```
Fat-free mass of whole body = weight - amount of body fat
of whole body
```

In conventional technology 1, about calculation of an above-mentioned body fat ratio, the body fat ratio of whole body was presumed by applying "left side of the body resistance" by measuring current and voltage of between left hand and left leg to regression for the whole body.
Thus, in case of a subject without one arm, one leg, both arms, or both legs, and a being-measured person who is in a plaster cast or the like by the injury and can not be touched his / her skin with all the electrodes, the device serves out the error display and has stopped because it is impossible to have computed the exact value.

Therefore, as a result of the artificer of this invention inquiring wholeheartedly, they are invented having two or more regression, which have been loaded only one formerly.

Here, formerly, it was thought that an error ratio increased and the reliability of measured value declined when two or more regression were loaded for each parts of the body.

Therefore, the inventors of this invention have experiment and research wholeheartedly about these errors, when two or more regression is loaded in each part of the body.

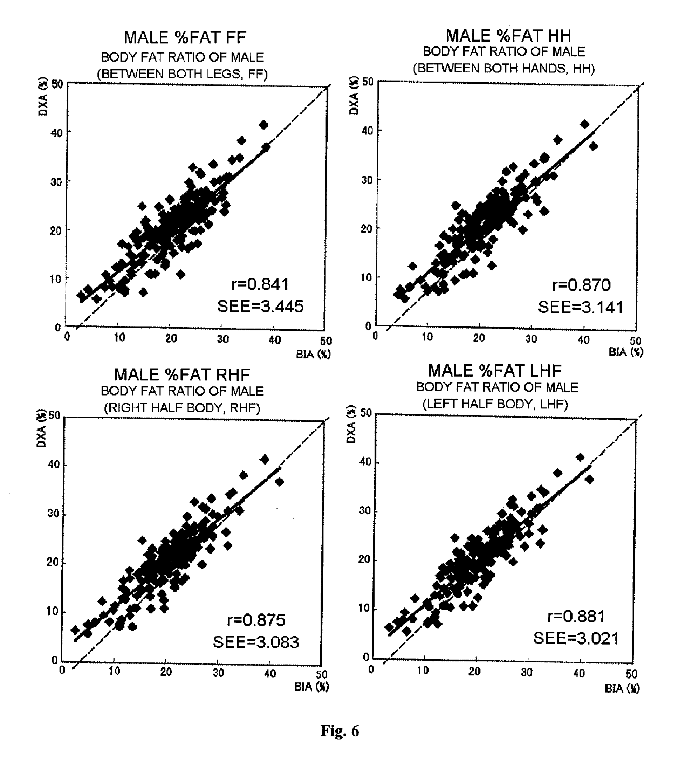

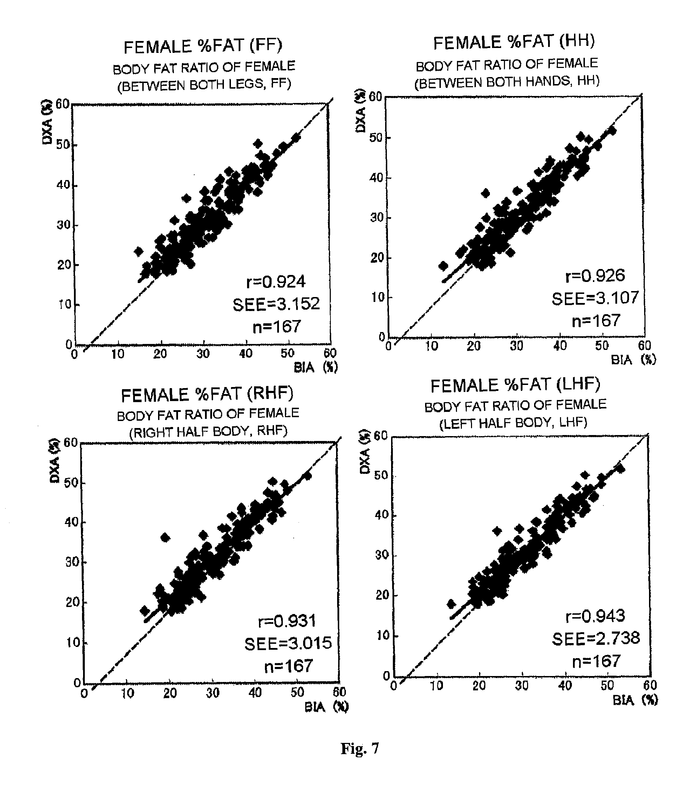

It explains with reference to Fig. 6 and Fig. 7 which are experimental results; about a being-measured person which is missing in each part of the body, a being-measured person fixed by a plaster cast, or a healthy person, respectively, a body fat ratio is computed from a value of bioelectricity impedance of each part of the body and is compared with a fat rate of the whole body determined by the DXA method.

Specifically, investigated about correlativity by being compared with a body fat ratio of whole body computed from a value of each bioelectricity impedance (horizontal axis,) which are between both feet (between both legs, FF), between both hands (HH, or between both arms), right half body (RHF) which is between right hand and right leg (between right arm and right foot), and left half body (LHF) which is between left hand and left leg (between left arm and left foot) for male in Fig. 6 and for female in Fig. 7, respectively, and a body fat ratio (vertical axis) of the whole body, which is determined by using the conventional DXA (Dual energy X-ray Absorptiometry) method for calculating composition of a human body from the transmission quantity of two kinds of radiation of the different wavelengths.

As a result, the value of the bioelectricity impedance of each part of the body to regression has extremely high correlativity. Thus, even if preparing a plurality of regression, it turned out that calculating accurately the amount of body fat of the whole body is enabled.

Therefore, in an embodiment of the invention, we decided to determine for a body fat ratio of the whole body about a being-measured person which cannot apply an electrode to each part of the body by using regressions, which are for between both feet (FF), for between both hands (HH), for left half bodies (LHF), and for right half bodies (RHF), respectively.

As such regression, the following equations can be prepared according to man and woman and can be used:

Body fat ratio of whole body = f (height, weight, resistance between both feet, reactance between both feet, and impedance between both feet) .... for FF

Body fat ratio of whole body = f (height, weight, resistance between both hands, reactance between both hands, and impedance between both hands) .... for HH

Body fat ratio of whole body = f (height, weight, resistance of left half body, reactance of left half body, and impedance of left half body) .... for LHF

Body fat ratio of whole body = f (height, weight, resistance of right half body, reactance of right half body, and impedance of right half body) .... for RHF

Similarly, we decided to determine for a body fat ratio of each part about a being-measured person who cannot apply an electrode to each part of the body by using regressions, which are for left leg (left foot, LF), for right leg (right foot, RF), for left arm (left hand, LH), for right arm (right hand RH), and for trunk (HT), respectively. As such regression, the following equations can be prepared according to man and woman and can be used:

Body fat ratio of left leg = f(height, weight, resistance of left leg, reactance of left leg, and impedance of left leg) .... for LF

Body fat ratio of right leg = f(height, weight, resistance of right leg, reactance of right leg, and impedance of right leg) .... for RF

Body fat ratio of left arm = f(height, weight, resistance of left arm, reactance of left arm, and impedance of left arm) .... for LH

Body fat ratio of right arm = f(height, weight, resistance of right arm, reactance of right arm, and impedance of right arm) .... for RH

Body fat ratio of trunk = f(height, weight, resistance of trunk, reactance of trunk, and impedance of trunk) .... for HT

About the amount of body fat of each part, it can be determined with the weight of each part for which is determined from weight by using the following equation.

```
    Amount of body fat of each part = weight of each part * body fat ratio of each part
```

By the above equation, the body composition monitor that is highly precise and can determine the body composition of the whole body and each part of the body when required about the being-measured person who cannot apply an electrode to each part of the body can be provided.

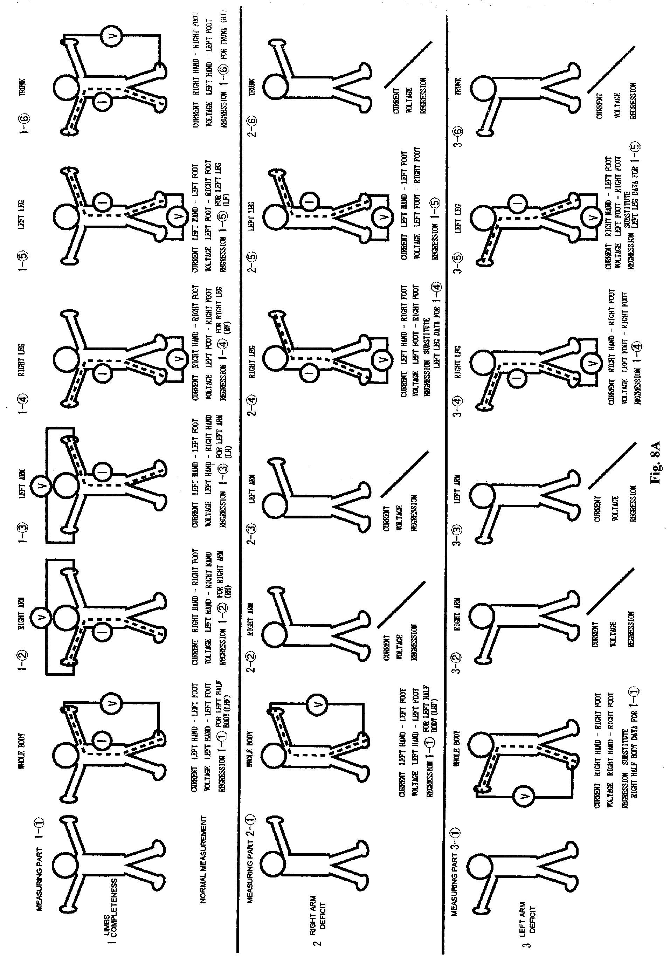

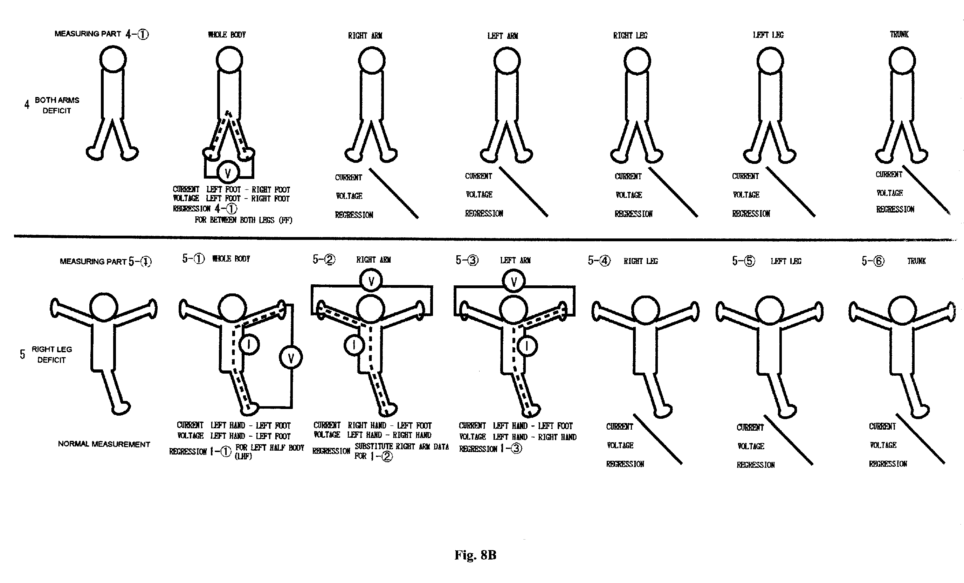

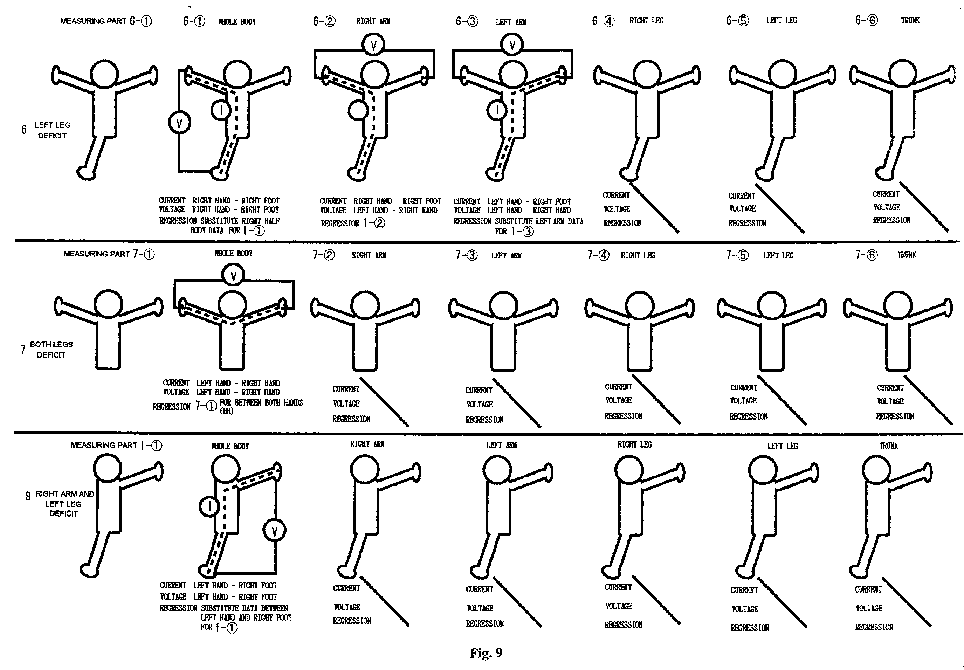

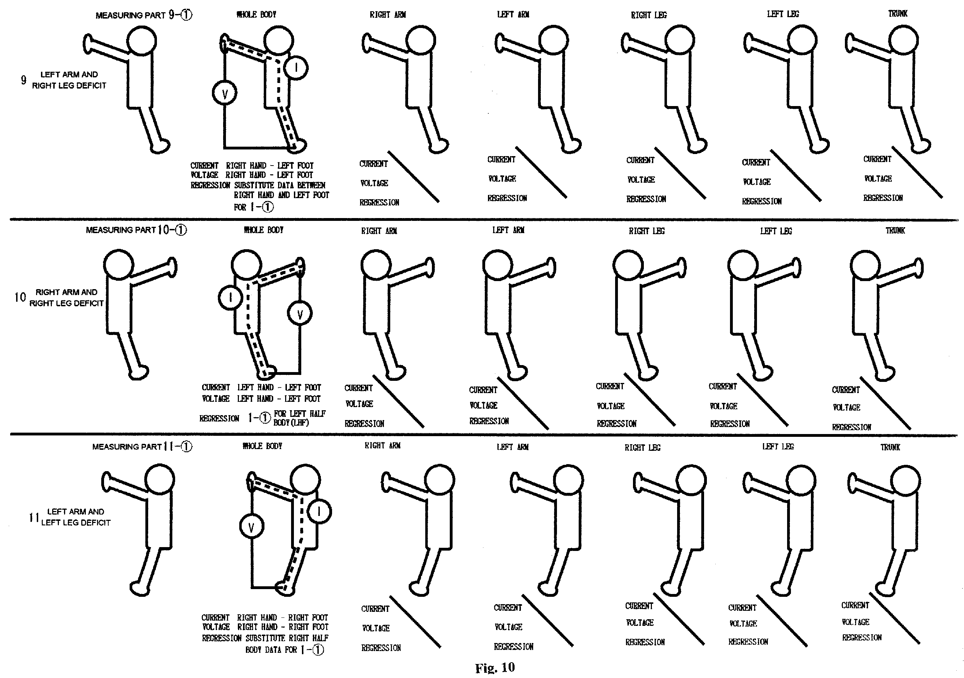

Subsequently, the case where a body fat ratio or the like are computed in the being-measured person who can not touch the skin with an electrodes by using the above-mentioned equation is explained in more detail, as refer to Figs. 8A - 10.

Figs. 8A - 10 are conceptual diagrams explaining a method of calculation of a body fat ratio about each case of 1. limbs completeness, 2. right arm deficit, 3. left arm deficit, 4. both-arms deficit, 5. right leg deficit, 6. left leg deficit, 7. both-legs deficit, 8. right arm and left leg deficit, 9. left arm and right leg deficit, 10. right arm and right leg deficit, and 11. left arm and left leg deficit, respectively.

In these figures, an electrode which makes the application of the current to each part, an electrode which measures voltage, and a regression used in order to measure a body fat ratio of each part of the body or the like are shown.

Hereafter, it explains in detail for every case more definitely.

[Case 1. limbs completeness]

A case where a body fat ratio is measured about a being-measured person of case 1, limbs completeness, that is, a being-measured person who can apply an electrode to each part of the whole body, is explained.

When measuring the whole body, in Step S104, current is applied between left hand and left foot and voltage between left hand and left foot is measured.

Namely, by electrode switching device 20, current is applied between current applicator 13a and 3a, and voltage between voltage measurement electrode 13b and electrode 3b is measured.

Moreover, values of data (height, weight, and resistance reactance impedance of between left hand and left leg) obtained at Step S102 - Step S104 to regression for LHF is assigned, and the body fat ratio of the whole body is measured.

Then, each part of the body is measured.

When measuring a right arm, in Step S104, current is applied between right hand and right foot, and voltage between left hand and right hand is measured.

Namely, by electrode switching device 20, current is applied between current applicator 14a and 4a, and voltage between voltage measurement electrodes 13b and 14b is measured.

Moreover, values of data (height, weight, and resistance reactance impedance of a right arm) obtained at Step S102 - Step S104 is assigned to regression for RH, and the body fat ratio of the right arm is measured.

When measuring the left arm, in Step S104, current is applied between left hand and left foot, and voltage between the left hand and right hand is measured. Namely, by electrode switching device 20, current is applied between current applicator 13a and 3a, and voltage between voltage measurement electrodes 13b and 14b is measured.

Moreover, values of data (height, weight, and resistance reactance impedance of the left arm) obtained at Step S102 - Step S104 is assigned to regression for LH, and the body fat ratio of the left arm is measured.

When measuring the right leg, in Step S104, current is applied between right hand and right foot, and voltage between left foot and right foot is measured.

Namely, by electrode switching device 20, current is applied among current applicators 14a and 4a, and voltage between voltage measurement electrodes 3b and 4b is measured.

Moreover, a value of data (height, weight, and resistance reactance impedance of a right leg) obtained at Step S102 - Step S104 is assigned to regression for RF, and the body fat ratio of the right leg is measured.

When measuring the left leg, in Step S104, current is applied between left hand and left foot, and voltage between left foot and right foot is measured.

Namely, by electrode switching device 20, current is applied among current applicators 13a and 3a, and voltage between voltage measurement electrodes 3b and 4b is measured.

Moreover, values of data (height, weight, resistance reactance impedance of a left leg) obtained at Step S102 - Step S104 is assigned to regression for LF, and the body fat ratio of the left leg is measured.

When measuring the trunk, in Step S104, current is applied between right hand and right foot, and voltage between left hand and left foot is measured.

Namely, by electrode switching device 20, current is applied among current applicators 14a and 4a, and voltage between voltage measurement electrodes 13b and 3b is measured.

Moreover, values of data (height, weight, resistance reactance impedance of a trunk) obtained at Step S102 - Step S104 is assigned to regression for HT, and the body fat ratio of the trunk is measured.

[Case 2. right arm deficit]

Then, measurement of the body fat ratio in the case 2, right arm deficit, is explained.

When measuring the whole body, it measures as well as a case of above-mentioned case 1.

Because a right arm cannot apply an electrode or it does not exist, it is not measured.

In this case, the left arm is not measured, because it is not applied to regression for LH.

When measuring a right leg, in Step S104, current is applied between left hand and right foot, and voltage between left foot and right foot is measured.

Namely, by electrode switching device 20, current is applied among current applicators 13a and 4a, and voltage between voltage measurement electrodes 3b and 4b is measured.

Moreover, values of data (height, weight, and resistance reactance impedance of a right leg) obtained at Step S102 - Step S104 are substitute to regression for RF, and the body fat ratio of the right leg is measured.

This is, even if applying current between left hand and right foot, a value of measured voltage becomes almost the same in the case of applying between right hand and right foot.

When measuring a left leg, it measures as well as above-mentioned case 1.

The trunk is not measured because it can not be applied to regression for HT.

[Case 3. left arm deficit]

Then, measurement of a body fat ratio in the case 3, left arm deficit, is explained.

When measuring the whole body, in Step S104, current is applied between right hand and right foot, and voltage between right hand and right foot is measured.

Namely, by electrode switching device 20, current is applied among current applicators 14a and 4a, and voltage between voltage measurement electrodes 14b and 4b is measured.

Moreover, values of data (height, weight, resistance reactance impedance of the right half body) obtained at Step S102 - Step S104 is substituted for regression of LHF, and the body fat ratio of the whole body is measured.

This is because regression for RHF becomes almost the same as regression for LHF.

The right arm is not measured because it cannot be applied to regression for RH.

Because the left arm cannot apply an electrode or does not exist, it is not measured.

When measuring the right leg, it measures as well as above-mentioned case 1.

When measuring a left leg, in Step S104, current is applied between right hand and left foot, and voltage between left foot and right foot is measured.

Namely, by electrode switching device 20, current is applied among current applicators 14a and 3a, and voltage between voltage measurement electrode 3b and electrode 4b is measured.

Moreover, values of data (height, weight, and resistance reactance impedance of a left leg) obtained at Step S102 - Step S104 is substituted for regression of LF, and a body fat ratio of a left leg is measured.

This is, even if applying current between right hand and left foot, a value of measured voltage become almost the same as the case of applying current between right hand and right foot.

The trunk is not measured because it cannot be applied to regression for HT.

[Case 4. both arms deficit]

Then, measurement of a body fat ratio in the case 4, both-arms deficit is explained.

When measuring the whole body, in Step S104, current is applied between left foot and right foot, and voltage between left foot and right foot is measured.

Namely, by electrode switching device 20, current is applied among current applicators 3a and 4a, and voltage between voltage measurement electrode 3b and electrode 4b is measured.

Moreover, values of data (height, weight, and resistance reactance impedance of between both feet) obtained at Step S102 - Step S104 is substituted for regression of FF, and the body fat ratio of the whole body is measured.

Other values are not measured because the right arm and the left arm do not exist.

[Case 5. right leg deficit]

Then, measurement of a body fat ratio in the case 5, right leg deficit is explained.

When measuring the whole body, it measures as well as above-mentioned case 1.

When measuring a right arm, in Step S104, current is applied between right hand and left foot, and voltage between left hand and right hand is measured.

Namely, by electrode switching device 20, current is applied between current applicators 14a and 3a, and voltage between voltage measurement electrode 13b and electrode 14b is measured.

Moreover, value of data (height, weight, and resistance reactance impedance of a right arm) obtained at Step S102 - Step S104 is substituted to regression for RH, and the body fat ratio of the right arm is measured.

This is, even if applying current between right hand and left foot, a value of measured voltage becomes almost the same as the case of applying between right hand and right foot.

When measuring the left arm, it measures as well as above-mentioned case 1.

Because the right leg cannot apply an electrode or it does not exist, it is not measured.

The left leg is not measured because it is inapplicable to regression for LF.

The trunk is not measured because it cannot be applied to regression for HT.

[Case 6. left leg deficit]

Then, measurement of a body fat ratio in the case 6, left leg deficit, is explained.

When measuring the whole body, it measures as well as above-mentioned case 3.

When measuring a right arm, it measures as well as above-mentioned case 1.

When measuring the left arm, in Step S104, current is applied between left hand and right foot, and voltage between left hand and right hand is measured.

Namely, by electrode switching device 20, current is applied among current applicators 13a and 4a, and voltage between voltage measurement electrode 13b and electrode 14b is measured.

Moreover, values of data (height, weight, and resistance reactance impedance of the left arm) obtained at Step S102 - Step S104 is assigned to regression for LH, and the body fat ratio of the left arm is measured.

This is, even if applying current between left hand and right foot, a value of measured voltage become almost the same in the case where applying current between left hand and left foot.

The right leg is not measured because it is inapplicable to regression for RF.

Because the left leg cannot apply an electrode or it does not exist, it is not measured.

The trunk is not measured because it cannot be applied to regression for HT.

[Case 7. both legs deficit]

Then, measurement of a body fat ratio in the case 7, both-legs deficit, is explained.

When measuring the whole body, in Step S104, current is applied between left hand and right hand, and voltage between left hand and right hand is measured.

Namely, by electrode switching device 20, current is applied among current applicators 13a and 14a, and voltage between voltage measurement electrode 13b and electrode 14b is measured.

Moreover, the values of data (height, weight, resistance reactance impedance of between both hands) obtained at Step S102 - Step S104 are assigned to regression for HH, and the body fat ratio of the whole body is measured.

Other values are not measured because the right leg and the left leg do not exist.

[Case 8. right arm and left leg deficit]

Then, measurement of a body fat ratio in the case 8, right arm and a left leg deficit, is explained.

When measuring the whole body, in Step S104, current is applied between left hand and right foot, and voltage between left hand and right foot is measured.

Namely, by electrode switching device 20, current is applied among current applicators 13a and 4a, and voltage between voltage measurement electrode 13b and electrode 4b is measured.

Moreover, values of data (height, weight, and resistance reactance impedance between left hand and right foot) obtained at Step S102 - Step S104 is assigned to regression for LHF, and the body fat ratio of the whole body is measured.

As mentioned above, since measured value at in case of changing a right foot and a left foot is almost the same, it can also be calculated in this way.

Other values are not measured because right arm and left leg do not exist.

[Case 9. left arm and right leg deficit]

Then, measurement of a body fat ratio in the case 9, left arm and right leg deficit, is explained.
When measuring the whole body, in Step S104, current is applied between right hand and left foot, and voltage between right hand and left foot is measured.
Namely, by electrode switching device 20, current is applied among current applicators 14a and 3a, and voltage between voltage measurement electrode 14b and electrode 3b is measured.
Moreover, values of data (height, weight, and resistance reactance impedance between right hand and left foot) obtained at Step S102 - Step S104 is assigned to regression for LHF, and the body fat ratio of the whole body is measured.
As mentioned above, since measured value at in case of changing a left hand and a right hand is almost the same, it can also be assigned in this way.
Other values are not measured because left arm and right leg do not exist.

[Case 10. right arm and right leg deficit]

Then, measurement of a body fat ratio in the case of a case 10, right arm and right leg deficit, is explained.
When measuring the whole body, it measures as similar to the above-mentioned case 1.
Other values are not measured because right arm and right leg do not exist.

[Case 11. left arm and left leg deficit]

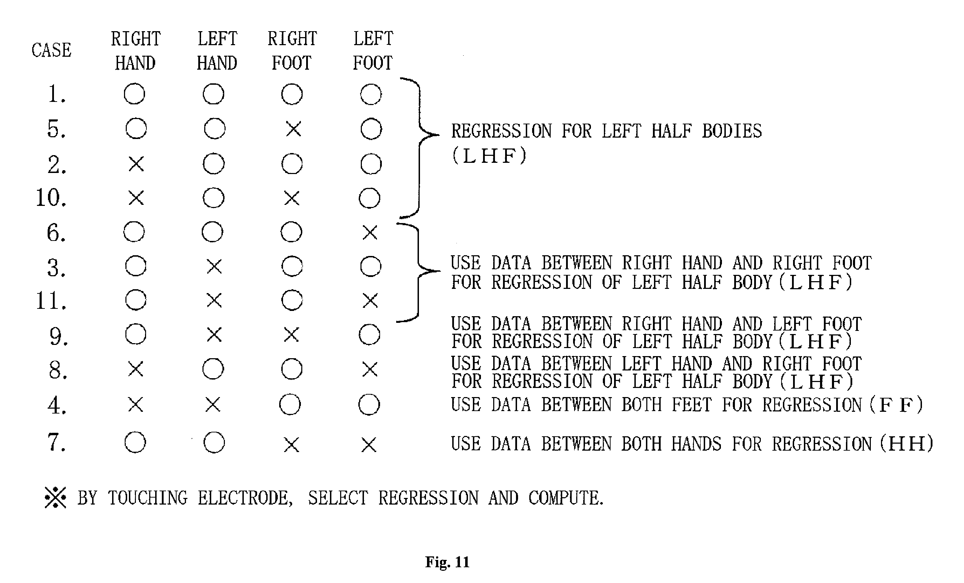

Then, measurement of a body fat ratio in the case of a case 11, left arm and left leg deficit, is explained.
When measuring the whole body, it measures as similar to the above-mentioned case 3.
Other values are not measured because left arm and left leg do not exist.
As mentioned above, by measuring each regression used in various kinds of cases, values about body composition including a body fat ratio of whole body and each part of the body can be calculated as much as possible for being-measured person who cannot put some electrodes to each part of the body.
In each case, as refer to figure 11 indicating which regression is mainly used, even if it cannot apply an electrode to one place or two places of right hand, right foot, left hand, and left foot, it turns out that it enables to apply to one of regression and cope with by preparing two or more regression.
The marks, O (applicable) and x (not-applicable) in fig. 11 indicate applicability of an electrode to the respective part.
For example, cases 8 is right arm and left leg deficit and do not have an electrode applied to this part as mentioned above; therefore, it can determine for a body fat ratio of the whole body, or the like by applying measurement data of left arm and right leg to the regression for left half bodies.

(Step S106)

Here, it returns and explains to a flow chart of Fig. 3. In Step S106, arithmetic and control unit 25 displays on display 7 that the measurement and operation are completed.

(Step S107)

Then, in Step S107, arithmetic and control unit 25 displays the values about body composition of the whole body and each part of the body on display 7 and carries out advice to the subject.
When measuring person make push-down of the key of input device 6, it is also enabled to display a targeted value about body composition (for example, body fat ratio) calculated with the individual parameter of being-measured person.
Further, when measuring person make push-down of the key of input device 6, advice of a measurement result can be displayed.
About this advice, it displays based on threshold values and texts memorized in ROM of memory storage 24.
Furthermore, when measuring person make push-down of the key of input device 6, arithmetic and control unit 25 prints the above-mentioned measurement result to printing device 8.
At the time of this printing, it is also enabled to print a document of more detailed advice.
These measurement results can be transmitted to the other computers by using communication apparatus 60.
In this case, checking about the measurement results are enabled on a screen of the other computers.
When the measurement results are confirmed, measuring person will turn off this measuring device 1, and will end measurement.
The following effects can be acquired with comprising as mentioned above:

Firstly, in conventional technology 1, unless it applied all electrodes to right hand, right foot, left hand, and left foot, measurement of body composition was not completed.

However, in measuring device 1 related to an embodiment of the invention, when an electrode is not applied to any one in right hand, right foot, left hand, and left foot, it is enabled to compute a value about body composition of whole body and a value about body composition of parts other than a part to which the electrode cannot be applied.

Hence, even if there is not one leg, there is not one arm, and it cannot grasp an electrode grip, measurement can be done. The measurement becomes enabled also about a person of hemiplegia, a person of cutting an arm or a leg in the middle, and a person who is physically handicapped.

When electrodes are not applied to any two in right hand, right foot, left hand, and left foot, it is enabled at least to compute the value about body composition of whole body.

Thereby, dissatisfaction for being-measured person who was not able to measure body composition by conventional technology 1 until now is cancelable.

Also, measuring device 1 related to an embodiment of the invention can measure a value about body composition according to physical action by the physically handicapped person.

A value about body composition of the whole body can be measured also about the athlete or the like who fixed in a plaster cast by an accident or the like, which makes impossible to measure body composition.

In addition, it is enabled to use for health care administration in a case of performing rehabilitation for, such as, an accident.

A healthy person who can apply an electrode to the limbs may also be unable to measure correctly by a measuring device of conventional technology 1, wherein the case that the skin of a leg has keratinized, strength to grasp is weak and contact with an electrode grip is not enough, contact resistance with an electrode has fallen remarkably with sweat, or the like.

However, by preparing a plurality of regression such as measuring device 1 related to an embodiment of the invention, an error display can be displayed as compared with a value about body composition determined from another regression.

It is also enabled to set up compute a value about body composition only excluding an abnormal value.

Furthermore, when a value about body composition in comparison with a value about body composition determined from other regression is normal, and more reliable measurement of body composition can be performed by averaging with these values as compared with conventional technology 1.

When measuring device 1 related to an embodiment of the invention can measure body composition such as an above-mentioned example, in the case of right arm deficit, in the case of left arm deficit, in the case of both-arms deficit, in the case of right leg deficit, in the case of left leg deficit, in the case of both-legs deficit, in the case of right arm and left leg deficit, in the case of left arm and right leg deficit, in the case of a right arm and right leg deficit, and in the case of the left arm and left leg deficit, are included; however, it is not restricted to these cases.

For example, even when there are not both arms and both legs, it can be measured exact body composition by designing regression to apply an electrode to the abdominal or the parietal region and to apply an electrode among other parts.

Furthermore, in the case of being-measured person who has lost the bottom from some joints of an arm or a leg, it is enabled to add an compensation to regression and to cope with it by designating a position to which an electrode is put.

As an example of above-mentioned body composition, a body fat ratio, amount of body fat, a fat-free rate, and a fat-free mass are mainly explained; however, in addition, for example, by measuring muscular volume, condition of muscles strain for each part, balance of muscular volume, or the like, can be realized.

Although measuring device 1 related to an embodiment of the invention is explained in which measuring person measures bioelectricity impedance in a standing position with electrodes of the sole and electrodes to grasp, it is not restricted to this.

For example, it is also enabled to measure by sticking an electrode on each part of the body in the state where a subject lies.

Thereby, it becomes enabled to measure bioelectricity impedance smoothly also about a being-measured person who has injury in the leg.

Furthermore, unlike conventional technology 1, in measuring device 1 related to an embodiment of the invention, using input device 6 directly can input weight, and a measuring method of a body fat ratio which does not use a value of weight of a being-measured person can be employed.

Since this measuring device 1 could transmit a measurement result to the other computers and the other computers are provided with nonvolatile memory measures, such as HDD, which is an auxiliary storage, it can carry out investigating data of sequential observation conveniently.

Since measurement of body composition is convenient, it is suitable for pursuing a change with time of body composition; ascertainment visually because a numerical value increases with recovery from an injury or the like; and for this reason, it is enabled to promote volition applied to recovery of a measuring person or a being-measured person.

In addition, unlike a bioelectricity impedance measuring device of conventional technology 1, this measuring device 1 has memorized regression of a subject who includes a child and an old person in ROM of memory storage 24.

Therefore, it can measure accurately also about muscular volume of a child or an old person.

Consequently, it is enabled understand a reintegration degree more accurately.

Composition and operation of the above-mentioned embodiment are an example, and it cannot be it needless to say that it can change suitably and can execute in the range, which does not deviate from the purport of this invention.

**Claims**

1. A body composition measuring device providing

   a calculator determining body composition by bioelectricity impedance computed from measured value by using an electrode of current application and an electrode of voltage measurement, wherein the calculator determines body composition by using regression(s) of the bioelectricity impedance corresponding to each part of between both feet, between both arms, left half body, right half body, left leg, right leg, left arm, right arm, and trunk.

2. The body composition measuring device according to claim 1, wherein a plurality of the electrode of current application and the electrode of voltage measurements are provided for a being-measured person corresponding to the left arm, the right arm, the left leg, and the right leg.

3. The body composition measuring device according to claim 2, wherein:

   in the electrode of current application and the electrode of voltage measurement provided for the being-measured person corresponding to the left arm, the right arm, the left leg, and the right leg, using the other electrodes for the part of the bioelectricity impedance measurement, and measuring body composition by using the regression corresponding to an electrode which is not used.

4. The body composition measuring device according to claim 3, wherein:

   when not using an electrode corresponding to the right arm and / or the right leg, body composition is measured by using the regression corresponding to the left arm and / or a left leg.

5. The body composition measuring device according to claim 3, wherein:

   when not using an electrode corresponding to the left arm and / or a left leg, body composition is measured by using a value of the bioelectricity impedance measurement of an electrode corresponding to a right arm and / or a right leg for the regression corresponding to the left arm and / or a left leg.

6. The body composition measuring device according to claim 3, wherein:

   when not using electrodes corresponding to the left arm and the right leg, body composition is measured by using a value of the bioelectricity impedance measurement of electrodes corresponding to the right arm and the left leg for the regression corresponding to the left arm and the left leg.

7. The body composition measuring device according to claim 3, wherein:

   when not using electrodes corresponding to the right arm and the left leg, body composition is measured by using a value of the bioelectricity impedance measurement of electrodes corresponding to the left arm and the right leg for the regression corresponding to the left arm and the left leg.

8. The body composition measuring device according to any one of claims 1 to 7, wherein:

   carrying out equalization or removal of an abnormal value for a value of body composition determined from a plurality of the regression.

9. The body composition measuring device according to any one of claims 1 to 8, wherein:

   the body composition of a part of body by using an electrode is measured in addition to body composition of whole body.

**10.** A body composition measuring method comprising:

computing bioelectricity impedance from measured value by using an electrode of current application and an electrode of voltage measurement, and
determining body composition from the bioelectricity impedance, wherein
determining body composition by using regression(s) of the bioelectricity impedance corresponding to each part of between both feet, between both hands, right half body, left half body, right leg, left leg, right arm, left arm, and trunk.

**Fig. 1**

LEFT FOOT     RIGHT FOOT     LEFT HAND     RIGHT HAND

( 3a )  ( 3b )   ( 4a )  ( 4b )   ( 13a )  ( 13b )   ( 14a )  ( 14b )

ELECTRODE SWITCHING DEVICE

20

CURRENT SUPPLY
SOURCE DEVICE

21

VOLTAGE MEASURE-
MENT DEVICE

22

ARITHMETIC AND CONTROL UNIT

25

DISPLAY DEVICE     INPUT DEVICE

7                                6

PRINTING DEVICE     MEMORY STORAGE

8                                24

MEASUREMENT-OF-
BODY-WEIGHT DEVICE

26

COMMUNICATION
APPARATUS

60

ELECTRIC POWER
UNIT

28

**Fig. 2**

START

INITIALIZATION PROCESS — S101

PREPARE-FOR-MEASURMENT PROCESS — S102

MEASURE BODY WEIGHT — S103

MEASURE BIOELECTRICITY IMPEDANCE — S104

CALCULATE VALUES ABOUT BODY COMPOSITION — S105

DISPLAY MEASUREMENT AND OPERATION ARE COMPLETED — S106

DISPLAY BODY COMPOSITION AND ADVICE — S107

END

**Fig. 3**

Fig. 4

**Fig. 5**

Fig. 6

## FEMALE %FAT (FF)
### BODY FAT RATIO OF FEMALE (BETWEEN BOTH LEGS, FF)

r=0.924
SEE=3.152
n=167

## FEMALE %FAT (HH)
### BODY FAT RATIO OF FEMALE (BETWEEN BOTH HANDS, HH)

r=0.926
SEE=3.107
n=167

## FEMALE %FAT (RHF)
### BODY FAT RATIO OF FEMALE (RIGHT HALF BODY, RHF)

r=0.931
SEE=3.015
n=167

## FEMALE %FAT (LHF)
### BODY FAT RATIO OF FEMALE (LEFT HALF BODY, LHF)

r=0.943
SEE=2.738
n=167

**Fig. 7**

MEASURING PART 1-①    WHOLE BODY    1-② RIGHT ARM    1-③ LEFT ARM    1-④ RIGHT LEG    1-⑤ LEFT LEG    1-⑥ TRUNK

1 LIMBS COMPLETENESS

NORMAL MEASUREMENT

CURRENT LEFT HAND – LEFT FOOT
VOLTAGE LEFT HAND – LEFT FOOT
REGRESSION 1-① FOR LEFT HALF BODY (LHF)

CURRENT RIGHT HAND – RIGHT FOOT
VOLTAGE LEFT HAND – RIGHT HAND
REGRESSION 1-② FOR RIGHT ARM (RH)

CURRENT LEFT HAND – LEFT FOOT
VOLTAGE LEFT HAND – RIGHT HAND
REGRESSION 1-③ FOR LEFT ARM (LH)

CURRENT RIGHT HAND – RIGHT FOOT
VOLTAGE LEFT FOOT – RIGHT FOOT
REGRESSION 1-④ FOR RIGHT LEG (RF)

CURRENT LEFT HAND – LEFT FOOT
VOLTAGE LEFT FOOT – RIGHT FOOT
REGRESSION 1-⑤ FOR LEFT LEG (LF)

CURRENT RIGHT HAND – RIGHT FOOT
VOLTAGE LEFT HAND – LEFT FOOT
REGRESSION 1-⑥ FOR TRUNK (RF)

---

MEASURING PART 2-①    WHOLE BODY    2-② RIGHT ARM    2-③ LEFT ARM    2-④ RIGHT LEG    2-⑤ LEFT LEG    2-⑥ TRUNK

2 RIGHT ARM DEFICIT

CURRENT LEFT HAND – LEFT FOOT
VOLTAGE LEFT HAND – LEFT FOOT
REGRESSION 1-① FOR LEFT HALF BODY (LHF)

CURRENT
VOLTAGE
REGRESSION

CURRENT
VOLTAGE
REGRESSION

CURRENT LEFT HAND – RIGHT FOOT
VOLTAGE LEFT FOOT – RIGHT FOOT
REGRESSION SUBSTITUTE LEFT LEG DATA FOR 1-④

CURRENT LEFT HAND – LEFT FOOT
VOLTAGE LEFT FOOT – RIGHT FOOT
REGRESSION 1-⑤

CURRENT
VOLTAGE
REGRESSION

---

MEASURING PART 3-①    WHOLE BODY    3-② RIGHT ARM    3-③ LEFT ARM    3-④ RIGHT LEG    3-⑤ LEFT LEG    3-⑥ TRUNK

3 LEFT ARM DEFICIT

CURRENT RIGHT HAND – RIGHT FOOT
VOLTAGE RIGHT HAND – RIGHT FOOT
REGRESSION SUBSTITUTE RIGHT HALF BODY DATA FOR 1-①

CURRENT
VOLTAGE
REGRESSION

CURRENT
VOLTAGE
REGRESSION

CURRENT RIGHT HAND – RIGHT FOOT
VOLTAGE LEFT FOOT – RIGHT FOOT
REGRESSION 1-④

CURRENT RIGHT HAND – LEFT FOOT
VOLTAGE LEFT FOOT – RIGHT FOOT
REGRESSION SUBSTITUTE LEFT LEG DATA FOR 1-⑤

CURRENT
VOLTAGE
REGRESSION

**Fig. 8A**

Fig. 8B

MEASURING PART 6-① | 6-① WHOLE BODY | 6-② RIGHT ARM | 6-③ LEFT ARM | 6-④ RIGHT LEG | 6-⑤ LEFT LEG | 6-⑥ TRUNK

6 LEFT LEG DEFICIT

CURRENT RIGHT HAND - RIGHT FOOT
VOLTAGE RIGHT HAND - RIGHT FOOT
REGRESSION SUBSTITUTE RIGHT HALF BODY DATA FOR 1-①

CURRENT RIGHT HAND - RIGHT FOOT
VOLTAGE LEFT HAND - RIGHT HAND
REGRESSION 1-②

CURRENT LEFT HAND - RIGHT FOOT
VOLTAGE LEFT HAND - RIGHT HAND
REGRESSION SUBSTITUTE LEFT ARM DATA FOR 1-③

CURRENT
VOLTAGE
REGRESSION

CURRENT
VOLTAGE
REGRESSION

CURRENT
VOLTAGE
REGRESSION

MEASURING PART 7-① | WHOLE BODY | 7-② RIGHT ARM | 7-③ LEFT ARM | 7-④ RIGHT LEG | 7-⑤ LEFT LEG | 7-⑥ TRUNK

7 BOTH LEGS DEFICIT

CURRENT LEFT HAND - RIGHT HAND
VOLTAGE LEFT HAND - RIGHT HAND
REGRESSION 7-① FOR BETWEEN BOTH HANDS (HH)

CURRENT
VOLTAGE
REGRESSION

CURRENT
VOLTAGE
REGRESSION

CURRENT
VOLTAGE
REGRESSION

CURRENT
VOLTAGE
REGRESSION

CURRENT
VOLTAGE
REGRESSION

MEASURING PART 1-① | WHOLE BODY | RIGHT ARM | LEFT ARM | RIGHT LEG | LEFT LEG | TRUNK

8 RIGHT ARM AND LEFT LEG DEFICIT

CURRENT LEFT HAND - RIGHT FOOT
VOLTAGE LEFT HAND - RIGHT FOOT
REGRESSION SUBSTITUTE DATA BETWEEN LEFT HAND AND RIGHT FOOT FOR 1-①

CURRENT
VOLTAGE
REGRESSION

CURRENT
VOLTAGE
REGRESSION

CURRENT
VOLTAGE
REGRESSION

CURRENT
VOLTAGE
REGRESSION

CURRENT
VOLTAGE
REGRESSION

**Fig. 9**

Fig. 10

| CASE | RIGHT HAND | LEFT HAND | RIGHT FOOT | LEFT FOOT | |
|---|---|---|---|---|---|
| 1. | ○ | ○ | ○ | ○ | REGRESSION FOR LEFT HALF BODIES (L H F) |
| 5. | ○ | ○ | × | ○ | |
| 2. | × | ○ | ○ | ○ | |
| 10. | × | ○ | × | ○ | |
| 6. | ○ | ○ | ○ | × | USE DATA BETWEEN RIGHT HAND AND RIGHT FOOT FOR REGRESSION OF LEFT HALF BODY (L H F) |
| 3. | ○ | × | ○ | ○ | |
| 11. | ○ | × | ○ | × | |
| 9. | ○ | × | × | ○ | USE DATA BETWEEN RIGHT HAND AND LEFT FOOT FOR REGRESSION OF LEFT HALF BODY (L H F) |
| 8. | × | ○ | ○ | × | USE DATA BETWEEN LEFT HAND AND RIGHT FOOT FOR REGRESSION OF LEFT HALF BODY (L H F) |
| 4. | × | × | ○ | ○ | USE DATA BETWEEN BOTH FEET FOR REGRESSION (F F) |
| 7. | ○ | ○ | × | × | USE DATA BETWEEN BOTH HANDS FOR REGRESSION (H H) |

※ BY TOUCHING ELECTRODE, SELECT REGRESSION AND COMPUTE.

**Fig. 11**

WHOLE BODY    RIGHT LEG    LEFT LEG    RIGHT ARM    LEFT ARM

TRUNK

CURRENT

VOLTAGE

**Fig. 12**

**EUROPEAN SEARCH REPORT**

Application Number

EP 08 15 9566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/059242 A1 (MASUO YOSHIHISA [JP] ET AL) 25 March 2004 (2004-03-25) * abstract; figures 1,16,32 * * paragraphs [0001], [0025] - [0030], [0069], [0101] * * paragraphs [0162] - [0233] * * paragraphs [0241], [0242], [0248] * * paragraphs [0300] - [0365] * | 1-3,8-10 | INV. A61B5/053 |
| A | EP 1 203 562 A (TANITA SEISAKUSHO KK [JP]) 8 May 2002 (2002-05-08) * the whole document * | 1-10 | |
| A | WO 2005/051194 A (BIOSPACE CO LTD [KR]; CHA KI CHUL [KR]) 9 June 2005 (2005-06-09) * the whole document * | 1-10 | |
| A | WO 96/08198 A (CHA KI CHUL [KR]) 21 March 1996 (1996-03-21) * the whole document * | 1-10 | |

TECHNICAL FIELDS SEARCHED (IPC)

A61B

The present search report has been drawn up for all claims

| Place of search | Date of completion of the search | Examiner |
|---|---|---|
| Munich | 19 September 2008 | Daniel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 15 9566

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2008

| Patent document cited in search report | | Publication date | Patent family member(s) | | Publication date |
|---|---|---|---|---|---|
| US 2004059242 | A1 | 25-03-2004 | AU<br>CN<br>WO<br>JP | 1849602 A<br>1489447 A<br>0243586 A1<br>4124649 B2 | 11-06-2002<br>14-04-2004<br>06-06-2002<br>23-07-2008 |
| EP 1203562 | A | 08-05-2002 | CN<br>US | 1350830 A<br>2002052697 A1 | 29-05-2002<br>02-05-2002 |
| WO 2005051194 | A | 09-06-2005 | NONE | | |
| WO 9608198 | A | 21-03-1996 | NONE | | |

EPO FORM P0459

For more details about this annex : see Official Journal of the European Patent Office, No. 12/82

**EP 2 011 437 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007174699 A
- JP 2001178696 A